# EUROPEAN PATENT APPLICATION

(11) **EP 3 581 203 A1**
(43) Date of publication of application: **18.12.2019**
(21) Application number: 18177042.1
(22) Date of filing: 11.06.2018
(51) Int. Cl.: A61K 39/395, C07K 16/24, A61P 37/06, A61K 39/00

(54) **ANTIBODIES WITH INCREASED ACTIVITY IN THE DIGESTIVE TRACT**

(71) Applicant: Laboratoire Français du Fractionnement et des Biotechnologies, 91940 Les Ulis (FR)
(72) Inventor: GAUCHER, Christine, 59320 Sequedin (FR)
(74) Representative: IPAZ

(57) **Abstract**

The present invention concerns antibodies that are therapeutically effective in the digestive tract, due to their increased resistance to proteolysis by one or more digestive or gastrointestinal proteases.

## Description

### Technical field

The present invention relates to an antibody having an increased resistance to proteolysis by one or more digestive or gastrointestinal proteases, a method to obtain said antibody and use of this antibody in preventive and/or therapeutic treatment.

### Background of the invention

Antibodies (Abs) are ideal therapeutic agents for their specificity and flexibility. The antibody (Ab) targets a molecule, a cell or an organism through its binding to a specific epitope on an antigen mediated as dictated by the variable region of the antibody molecule. The antibody's specificity is complemented by its ability to mediate and/or initiate a variety of biological activities. For example, antibodies can modulate receptor-ligand interactions as agonists or antagonists. Antibody binding can initiate intracellular signaling to stimulate cell growth, cytokine production, or apoptosis. Antibodies deliver agents bound to the Fc region to specific sites. Antibodies also elicit antibody-mediated cytotoxicity (ADCC), complement-mediated cytotoxicity (CDC), and phagocytosis. Thus, antibodies are increasingly being used as therapeutic agents to treat various diseases.

While the properties of antibodies make them very attractive therapeutic agents, there are a number of limitations. One of the primary limitations is the stability of the antibody following administration to a subject. Antibodies are proteins and thus are susceptible to degradation by proteolytic enzymes present in, for example, the blood or digestive tract. Complete or partial degradation of the antibody prevents a therapeutically effective amount from reaching a distant target site.

Indeed, administration of antibodies in the digestive tract face an hurdle due to the presence of proteases. The stomach produces pepsin. The average person secretes about 400 mL of gastric fluid per meal, containing 50 to 300 µg pepsin/mL. The transit time in the stomach varies from 0.5 to 4.5 h. Protein digestion continues in the duodenum and jejunum (together called as small intestine), where proteolytic enzymes of the pancreas (trypsin, trypsinogen, chymo-trypsinogen, pro-carboxy-peptidase, and pro-elastase) attack the remaining breakdown products. The pH of the small intestine ranges from 6.3 to 7.5 with a transit time in the order of 1 to 4 h. In the colon, the pH is between 7.5 and 8 with a transit time of 8-16 h, creating a harsh environment for any orally administered protein.

Like most proteins, antibodies are degraded after oral administration. Antibodies are initially degraded into F(ab')2, Fab and Fc fragments. Even after this initial degradation, the F(ab')2 and Fab fragments retain some of their biological activity. For example, stool samples from adults receiving bovine milk immunoglobulin orally contained detectable amounts of antibody with neutralizing activity. However, degradation in these harsh conditions significantly limits the usefulness of orally administered antibodies.

This point is particularly highlighted in the publication of Yadav et al. (Yadav et al., International Journal of Pharmaceutics, 2016, vol 502, pages 181-187). In Yadav et al., the stability of infliximab and adalimumab (anti-TNF α antibodies) in human gastric fluid have been tested. Infliximab is a chimeric IgG1 kappa mAb composed of human constant region and murine variable region, produced and secreted by mouse myeloma cells (SP2/0 cells). Adalimumab, which was derived from phage display, is a recombinant fully human mAb with human derived variable regions and human IgG1 kappa constant regions, produced in Chinese hamster ovary cells. The authors have observed a rapid and extensive degradation of both antibodies in the digestive tract. In human intestinal fluid, 90% of the dose of infliximab was degraded within 30 min (t1/2 = 1.32 ±0.02 min). Infliximab followed a similar trend in simulated intestinal fluid with pancreatin, with 70% of the dose degraded within 30 min (t1/2 = 18.5±0.85 min).

Therefore, there is a need for the development of effective prophylactic and therapeutic antibodies orally administered and able to treat patients in need of, while simultaneously avoiding normal protease disruption. Thus, antibodies that retain biological activity following oral administration offer a significant improvement over the current prophylactic and therapeutic options currently available.

### Detailed description of the invention

The present invention concerns an antibody produced in mammary epithelial cells, wherein said antibody has an increased resistance to proteolysis by one or more digestive or gastrointestinal proteases.

The term "antibody" or "antibodies" or "population of antibodies" as used herein refers to immunoglobulin molecules and immunologically active portions of immunoglobulin molecules, i.e., molecules that contain an antigen binding site that specifically binds an antigen, whether natural or partly or wholly synthetically produced. The term also covers any polypeptide or protein having a binding domain which is, or is homologous to, an antibody binding domain. These can be derived from natural sources, or they may be partly or wholly synthetically produced. "antibody", "antibodies" and "population of antibodies" are used interchangeably herein.

An antibody is a roughly Y-shaped molecule composed of two different polypeptide chains named "heavy" and "light" chains, an antibody being constituted of two heavy and two light chains linked by disulfide bonds. Each heavy chain is composed of a variable domain ("VH domain") and 3 constant domains ("CH1", "CH2" and "CH3" domains), while each light chain is composed of a variable domain ("VL domain") and a constant domain ("CL domain"). In addition, each variable domain (VH or VL domain) is composed of 4 "framework regions" (FR1, FR2, FR3 and FR4), which display less variability among antibodies and are involved in the formation of beta sheets forming the structural framework of the variable domain, and of 3 hypervariable regions commonly named "complementary determining regions" 1, 2 and 3 (CDR1, CDR2, CDR3), which correspond to 3 loops juxtaposed in the folded variable domain at the edge of each beta sheet. The 3 CDR regions are crucial for determining an antibody or antibody fragment specificity, since they are the part of the variable domain mainly in contact with the antigen, especially the CDR3 region of each chain, which corresponds to the rearranged region of the heavy and light chains and is even more variable and more directly in contact with the specific antigen. Examples of antibodies are the immunoglobulin isotypes (e.g., IgG, IgE, IgM, IgD and IgA) and their isotypic subclasses. Antibodies may be polyclonal or monoclonal. A monoclonal antibody may be referred to herein as "mAb". In one embodiment, the antibody of the present invention is a monoclonal antibody.

As antibodies can be modified in a number of ways, the term "antibody" should be construed as covering any specific binding member or substance having a binding domain with the required specificity. Thus, this term covers antibody fragments, derivatives, functional equivalents and homologues of antibodies, humanized antibodies, including any polypeptide comprising an immunoglobulin binding domain, whether natural or wholly or partially synthetic. Chimeric molecules comprising an immunoglobulin binding domain, or equivalent, fused to another polypeptide are therefore included.

In one embodiment, the antibody of the present invention can be humanized, in particular by modifying the amino acid sequence of the antibody. Methods to reduce the immunogenicity of the specific binding molecules of the invention include CDR grafting on to a suitable antibody framework scaffold or variable surface residues remodeling, e.g. by site directed mutagenesis or other commonly used molecular biological techniques. A humanized antibody may be a modified antibody having the variable regions of a non-human, e.g. murine, antibody and the constant region of a human antibody. In one embodiment, the antibody of the present invention is a human antibody.

In another advantageous embodiment, the antibody of the invention can be a fragment of an antibody. It has been shown that fragments of a whole antibody can perform the function of binding antigens. Examples of binding fragments are:
(i) the Fab fragment consisting of VL, VH, CL and CHI domains;
(ii) the Fd fragment consisting of the VH and CHI domains;
(iii) the Fv fragment consisting of the VL and VH domains of a single antibody;
(iv) the dAb fragment, which consists of a V_{H} domain;
(v) isolated CDR regions;
(vi) F(ab')2 fragments, a bivalent fragment comprising two linked Fab fragments
(vii) single chain variable fragment molecules (scFv), wherein a VH domain and a VL domain are linked by a peptide linker which allows the two domains to associate to form an antigen binding site;
(viii) bispecific single chain Fv dimers and
(ix) "diabodies", multivalent or multispecific fragments constructed by gene fusion.
(x) scFv-Fc consisting in two scFv fragments with a Fc portion

As used herein, the term "CHI domain" includes the first (most amino terminal) constant region domain of an immunoglobulin heavy chain that extends, e.g., from about positions 114-223 in the Kabat numbering system (EU positions 118-215). The CHI domain is adjacent to the VH domain and amino terminal to the hinge region of an immunoglobulin heavy chain molecule, and does not form a part of the Fc region of an immunoglobulin heavy chain.

As used herein, the term "CL domain" comprises the constant region domain of an immunoglobulin light chain that extends, e.g. from about Kabat position 107A-216 (EU positions 108-214 (kappa)). The Eu/Kabat conversion table for the Kappa C domain is provided below. The CL domain is adjacent to the VL domain and includes the carboxy terminal of an immunoglobulin light chain.

In a preferred embodiment, the specific binding molecule of the invention is a single chain variable fragment (scFv) molecule. Within the context of the invention, the term "single chain variable fragment" or "scFv molecule" or "scFv fragment" or "scFv antibody" or "scFv" refers to a single folded polypeptide comprising the VH and VL domains of an antibody linked through a peptide linker. In such a scFv molecule, the VH and VL domains can be either in the VH-linker-VL or VL-linker-VH order. In addition to facilitate its production, a scFv molecule may contain a tag molecule linked to the scFv via a spacer. A scFv molecule thus comprises the VH and VL domains implicated into antigen recognizing but not the immunogenic constant domains of corresponding antibody.

The antibody of the invention may thus be produced by recombination in a mammary epithelial host cell, transformed with one or more vectors which allow the expression and/or the secretion of the nucleotide sequences coding for the heavy chain and/or the light chain of the antibody. The vector usually comprises a promoter, translation initiation and termination signals, as well as suitable transcription regulation regions. It is maintained in a stable manner in the host cell and may optionally have specific signals that specify the secretion of the translated protein. These various elements are selected and optimized by the person skilled in the art according to the host cell used. Such vectors are prepared by methods commonly used by the person skilled in the art, and the resulting clones may be introduced into a suitable host by standard methods, such as lipofection, electroporation, nucleofection, the use of gene gun, of polycationic agents, heat shock or chemical methods. The host cell may be selected from prokaryotic or eukaryotic systems, for example bacterial cells but also yeast cells or animal cells, in particular mammalian cells. In a particular embodiment of the invention, the antibody of the invention may be produced in mammary cells. In particularly advantageously embodiment, the antibody of the invention is produced in mammary epithelial cells of a non-human animal. As used herein, mammary epithelial cells of a non-human animal include primary cells, immortalized cell lines, and breast cancer cells having an epithelial origin. Breast cancer cells can be invasive or non-invasive. In yet another embodiment, the mammalian mammary epithelial cells of a non-human animal are mammalian mammary epithelial cells of a non-human animal in culture.

Another mode of production is the expression of the recombinant antibody in transgenic organisms, or particularly in the milk of transgenic animals such as rabbit, goat or pig. Transgenic milk can also be used as a method for the production of recombinant antibodies. In a particularly advantageous embodiment, the antibody of the invention is produced in the milk of a transgenic non-human animal.

The term "transgenic non-human animal" as used herein refers to a non-human mammals, genetically modified to produce this glycoprotein. The mammal may be for example a goat, a ewe, a female bison, a female buffalo, a female camel, a female llama, a mouse, a female rat, a cow, a sow, a doe, a female rabbit or a mare.

Secretion of the antibody by the mammary glands, allowing its presence in the milk of the transgenic mammal, involves tissue-dependent control of the expression of the antibody. Such methods of control are well-known to the person skilled in the art. Expression is controlled by means of sequences allowing expression of the glycoprotein in a particular tissue of the animal. They are in particular promotor sequences of the "WAP", "beta-casein", "betalactoglobulin" type and possibly sequences of the peptide signal type.

In advantageous embodiment of the invention, the antibody of the invention is produced in the mammary glands of a transgenic goat, using an expression vector comprising the sequence of the coding sequences needed for the production of the protein which are under the control of a 5' beta-casein promoter. A process for extracting proteins of interest from the milk of transgenic animals is described in the patent EP 0 264 166. In particularly embodiment of the present invention, the antibody of the invention is produced in the milk of a transgenic goat.

Advantageously, more than 4 grams of antibody per liter of milk is produced, advantageously more than 5, 10, 15, 20, 25, 30, 35 grams per liter.

Antibody glycosylation is a common post-translational modification and has a critical role in antibody effector function. Antibodies can be glycosylated with an N-glycan at the Fc-gamma glycosylation site in the heavy chain (Asn297) of the Fc region. Generally, antibodies include two heavy chains and each antibody therefore can have two Fc-gamma N-glycans. A variety of glycosylation patterns have been observed at the Fc gamma glycosylation site and the oligosaccharides found at this site include galactose, N-acetylglucosamine (GlcNac), mannose, sialic acid (N-acetylneuraminic acid (NeuAc or NANA), N-glycolylneuraminic (NGNA)) and fucose. N-glycans found at the Fc gamma glycosylation site generally have a common core structure consisting of an unbranched chain of a first N-acetylglucosamine (GlcNAc), which is attached to the asparagine of the antibody, a second GlcNAc that is attached to the first GlcNac and a first mannose that is attached to the second GlcNac. Two additional mannoses are attached to the first mannose of the GlcNAc-GlcNAc-mannose chain to complete the core structure and providing two "arms" for additional glycosylation. In addition, fucose residues may be attached to the N-linked first GlcNAc.

The two arm core structure is also referred to as the "antenna". The most common type of glycosylation of the "arms" of the N-glycan motifs found in plasma antibodies is of the complex type, i.e., consisting of more than one type of monosaccharide. In the biosynthetic route to this N-glycan motif, several GlcNAc transferases attach GlcNAc residues to the mannoses of the glycan core, which can be further extended by galactose and sialic acid residues. This glycosylation motif is called complex structure.

A second glycosylation motif found on the "arms" of the N-glycan core structure is a "high-mannose glycosylation pattern", which is characterized by additional mannoses (attached either as branched or unbranded chains).

A third glycosylation motif is a "hybrid structures" in which one of the arms is mannose substituted while the other arm is complex.

Within the context of the invention, the glycosylation motif of the antibody of the invention or population of antibodies of the invention is expressed by comparison to the total of glycan forms bound to N-glycosylation sites of said antibody or population of antibodies, obtained from the same sample of antibodies produced in mammary epithelial cells of a non-human animal or in the milk of a transgenic non-human animal.

In a particularly advantageous embodiment of the invention, the antibody or the population of antibodies of the invention, which is produced in mammary epithelial cells of a non-human animal or in the milk of a transgenic non-human animal, exhibits a high ratio galactose/fucose. Within the context of the invention, a "high ratio galactose/fucose" refers to a ratio galactose/fucose which is higher than 0,50. Advantageously, the ratio galactose/fucose of the antibody or the ratio galactose/fucose of the population of antibodies of the invention is higher than 0,55, advantageously higher than 0,60, advantageously higher than 0,65, advantageously higher than 0,70, advantageously higher than 0,75, advantageously higher than 0,80, advantageously higher than 0,85, advantageously higher than 0,90, advantageously higher than 0,95, advantageously higher than 1,00, advantageously higher than 1,05, advantageously higher than 1,10, advantageously higher than 1,15. In another embodiment of the invention, the ratio galactose/fucose of the antibody or the ratio galactose/fucose of the population of antibodies of the invention is less than 1,20. In another embodiment of the invention, the ratio galactose/fucose of the antibody or the ratio galactose/fucose of the population of antibodies of the invention is comprised between 0,50 and 1,20, advantageously between 0,70 and 1,15, advantageously between 0,75 and 1,15, more advantageously between 0,80 and 1,15.

In a particularly advantageous embodiment of the invention, the antibody or the population of antibodies of the invention, which is produced in mammary epithelial cells of a non-human animal or in the milk of a transgenic non-human animal, exhibits a low level of fucose.

Within the context of the invention, a "low level of fucose" refers to a level of fucose of the antibody or the level of fucose of the population of antibodies of the invention which is less than 50%. Advantageously, the level of fucose of the antibody or the level of fucose of the population of antibodies of the invention is less than 45%, advantageously less than 40%, advantageously less than 35%, advantageously less than 30%, advantageously less than 25%. In another embodiment of the invention, the level of fucose of the antibody or the level of fucose of the population of antibodies of the invention is not higher than 50%. In another embodiment of the invention, the level of fucose of the antibody or the level of fucose of the population of antibodies of the invention is comprised between 25% and 50%, advantageously between 30% and 50%, advantageously between 30% and 45%, more advantageously between 30% and 40%. In one embodiment, the fucose is 1,6-fucose.

In a particularly advantageous embodiment of the invention, the antibody or the population of antibodies of the invention, which is produced in mammary epithelial cells of a non-human animal or in the milk of a transgenic non-human animal, exhibits a low level of complex structures. Within the context of the invention, a "low level of complex structures" refers to a level of complex structures of the antibody or the level of complex structures of the population of antibodies of the invention which is less than 50%. Advantageously, the level of complex structures of the antibody or the level of complex structures of the population of antibodies of the invention is less than 47%, advantageously less than 45%, advantageously less than 40%, advantageously less than 35%, advantageously less than 30%, advantageously less than 25%, advantageously less than 20%. In another embodiment of the invention, the level of complex structures of the antibody or the level of complex structures of the population of antibodies of the invention is not higher than 50%. In another embodiment of the invention, the level of complex structures of the antibody or the level of complex structures of the population of antibodies of the invention is comprised between 19% and 50%, advantageously between 20% and 50%, advantageously between 20% and 45%, more advantageously between 20% and 40%.

In a particularly advantageous embodiment of the invention, the antibody or the population of antibodies of the invention, which is produced in mammary epithelial cells of a non-human animal or in the milk of a transgenic non-human animal, exhibits a high mannose glycosylation pattern and high hybrid structures.

Within the context of the invention, a "high mannose glycosylation pattern" is intended to refer to an antibody that contains at least one oligomannose or a population of antibodies, wherein at least 23% of the antibodies contain at least one oligomannose. In some embodiments at least 20%, 25%, 30%, 35%, 40%, 45%, 50%, 55%, 60%, 65% or more of the carbohydrates of the antibodies are oligomannose.

Within the context of the invention, a "high hybrid structure" is intended to refer to an antibody that contains at least one hybrid structure or a population of antibodies, wherein at least 16% of the antibodies contain at least one hybrid structure. In some embodiments at least 20%, 25%, 30%, 35 or more of the carbohydrates of the antibodies are hybrid structures.

Advantageously, the level of high mannose glycosylation pattern and hybrid structures of the antibody or the level of high mannose glycosylation pattern and hybrid structures of the population of antibodies of the invention is higher than 50%. Advantageously, the level of high mannose glycosylation pattern and hybrid structures of the antibody or the level of high mannose glycosylation pattern and hybrid structures of the population of antibodies of the invention is higher than 55%, advantageously higher than 60%, advantageously higher than 65%, advantageously higher than 70%, advantageously higher than 75%, advantageously higher than 80%, advantageously higher than 85%. In another embodiment of the invention, the high mannose glycosylation pattern and hybrid structures level of the antibody or the level of high mannose glycosylation pattern and hybrid structures of the population of antibodies of the invention is less than 90%. In another embodiment of the invention, the level of high mannose glycosylation pattern and hybrid structures of the antibody or the level of high mannose glycosylation pattern and hybrid structures of the population of antibodies of the invention is comprised between 50% and 85%, advantageously between 55% and 85%, advantageously between 60% and 85%, more advantageously between 65% and 85%, advantageously between 70% and 85%, more advantageously between 75% and 85%.

In a particularly advantageous embodiment of the invention, the antibody or the population of antibodies of the invention, which is produced in mammary epithelial cells of a non-human animal or in the milk of a transgenic non-human animal, is partially sialylated. The sialylation levels of such antibodies can be increased for instance by subjecting the antibodies to sialyltransferases. Advantageously, the sialylation level of the antibody or the sialylation level of the population of antibodies of the invention is higher than 17%. Advantageously, the sialylation level of the antibody or of the population of antibodies of the invention is higher than 18%, advantageously higher than 20%, advantageously higher than 25%, advantageously higher than 30%, advantageously higher than 35%, advantageously higher than 40%, advantageously higher than 45%. In another embodiment of the invention, the sialylation level of the antibody of the invention or the sialylation level of the population of antibodies of the invention is less than 50%. In another embodiment of the invention, the sialylation level of the antibody or the sialylation level of the population of antibodies of the invention is comprised between 17% and 50%, advantageously between 17% and 50%, advantageously between 20% and 50%, more advantageously between 30% and 45%.

In a particularly embodiment of the invention, the sialic acids of the antibody of the invention are bound in α2,6.

In a particularly advantageous embodiment of the invention, the antibody or the population of antibodies of the invention, which is produced in mammary epithelial cells of a non-human animal or in the milk of a transgenic non-human animal, is galactosylated. A "galactosylated" antibody, as used herein, refers to any antibody that has at least one galactose monosaccharide in one of its N-glycans. Galactosylated antibodies include antibodies where the two N-glycans each have complex type motifs on each of the arms of the N-glycan motifs, antibodies where the two N-glycans have a complex type motif on only one of the arms of the N-glycan motifs, antibodies that have one N-glycan with complex type motifs on each of the arms of the N-glycan, and antibodies that have one N-glycan with a complex type motif on only one of the arms of the N-glycan motifs. Antibodies that include at least one galactose monosaccharide include antibodies with N-glycans such as Gl (one galactose), GIF (one galactose, one fucose), G2 (two galactoses) and G2F (two galactoses, one fucose). In addition, the N-glycan that includes at least one galactose monosaccharide can be sialylated or not sialylated. It should further be appreciated that the N-glycans may also contain additional galactose residues, such as alpha-Gal, in one or more arms of the complex glycan motif, potentially resulting in an N-glycan with four galactose moieties.

Advantageously, the galactosylation level of the antibody or the galactosylation level of the population of antibodies of the invention is higher than 19%. Advantageously, the galactosylation level of the antibody or the galactosylation level of the population of antibodies of the invention is higher than 20%, advantageously higher than 25%, advantageously higher than 30%, advantageously higher than 35%, advantageously higher than 40%, advantageously higher than 45%, advantageously higher than 50%. In another embodiment of the invention, the galactosylation level of the antibody or the galactosylation level of the population of antibodies of the invention is less than 60%. In another embodiment of the invention, the galactosylation level of the antibody or the galactosylation level of the population of antibodies of the invention is comprised between 19% and 60%, advantageously between 20% and 60%, advantageously between 30% and 60%, more advantageously between 30% and 50%.

In a particularly advantageous embodiment of the invention, the antibody or the population of antibodies of the invention has an increased level of antibody-dependent cellular cytotoxicity (ADCC) activity when compared to antibodies not produced in mammary gland epithelial cells or in the milk of a transgenic non-human animal.

The term "increased resistance to proteolysis by one or more digestive or gastrointestinal proteases of an antibody of the invention" as used herein refers to comprises a greater resistance to proteolysis relative to a chimeric or human monoclonal antibodies produced in non-epithelial mammary cells. The term "non-epithelial mammary cells" refers for example to rat line YB2/0, the hamster line CHO, in particular the lines CHO dhfr- and CHO Lec13, PER.C6™, EB66, HEK293, K562, NS0, SP2/0, BHK or COS, the list is not limitative.

The specific glycosylation of the antibody or of the population of antibodies of the invention confers to the antibody or to the population of antibodies an increased resistance to proteolysis, when the antibody or the population of antibodies is administrated orally. In an advantageous embodiment of the invention, the high ratio galactose/fucose is responsible for the increased resistance to proteolysis of the antibody or of the population of antibodies of the invention.

In another advantageous embodiment of the invention, the specific combination of the high ratio galactose/fucose and the low level of fucose is responsible for the increased resistance to proteolysis of the antibody or of the population of antibodies of the invention.

In another advantageous embodiment of the invention, the specific combination of the high ratio galactose/fucose, the low level of fucose and the low level of complex forms is responsible for the increased resistance to proteolysis of the antibody or of the population of antibodies of the invention.

In another advantageous embodiment of the invention, the specific combination of the high ratio galactose/fucose, the low level of fucose, the low level of complex forms and the high level of hybrid and high mannose forms is responsible for the increased resistance to proteolysis of the antibody or of the population of antibodies of the invention.

In another advantageous embodiment of the invention, the specific combination of the high ratio galactose/fucose, the low level of fucose, the low level of complex forms, the high level of hybrid and high mannose forms and the partial sialylation of the antibody of the invention is responsible for the increased resistance to proteolysis of the antibody or of the population of antibodies of the invention.

In a particularly advantageous embodiment of the invention, the specific combination of the high ratio galactose/fucose, the low level of fucose, the low level of complex forms, the high level of hybrid and high mannose forms, the partial sialylation and the galactosylation of the antibody or of the population of antibodies of the invention is responsible for the increased resistance to proteolysis of the antibody of the invention.

In a particularly advantageous embodiment of the invention, the antibody or the population of antibodies of the invention exhibits:
- a high ratio galactose/fucose,
- a low level of fucose,
- a low level of complex forms,
- a high level of hybrid and high mannose forms,
- a partial sialylation, and
- a galactosylation.

Advantageously, the antibody the population of antibodies of the invention exhibits the following glycosylation pattern:
- a ratio galactose/fucose, which is higher than 0,5,
- a level of fucose, which is which is less than 50%,
- a level of complex forms, which is less than 50%,
- a level of hybrid structures and high mannose forms, which is higher than 50%,
- a level of sialylation, which is higher than 17%, and
- a level of galactosylation, which is higher than 19%.

Advantageously, the antibody the population of antibodies of the invention exhibits the following glycosylation pattern:
- a ratio galactose/fucose, which is comprised between 0,70 et 1,20,
- a level of fucose, which is comprised between 25% and 45%,
- a level of complex forms, which is comprised between 19% and 50%
- a level of hybrid structures and high mannose forms, which is comprised between 50% and 85%
- a level of sialylation, which is comprised between 17% and 45%, and
- a level of galactosylation, which is comprised between 19% and 50%.

The increased resistance to proteolysis can be at least about 1%, 2%, 3%, 4%, 5%, 10%, 15%, 20%, 25%, 30%, 40%, 50%, 60%, 70%, 80% or more than that of a chimeric or human monoclonal antibodies or a population of such antibodies produced in non-epithelial mammary cells. The antibody of the invention produced in mammary epithelial cells, advantageously in mammary epithelial cells of a non-human animal or in the milk of a transgenic non-human animal, can be partially or completely resistant to cleavage by more than one protease.

In some embodiments, the proteolysis inhibited by the antibody of the invention includes digestion mediated by proteases from the gastrointestinal track, the blood, or the bile. In advantageous embodiment of the invention, the antibody or the population of antibodies of the invention has an increased resistance to proteolysis more than one digestive or gastrointestinal proteases.

Within the context of the invention, the term "digestive or gastrointestinal proteases" refers to pepsin, pancreatin, trypsin, chymotrypsin, carboxypepsidase, procarboxypepsidase, elastase, proelastase or a combination thereof.

Advantageously, the antibody or the population of antibodies of the invention has an increased resistance to proteolysis by pepsin, pancreatin, trypsin, chymotrypsin, carboxypepsidase, procarboxypepsidase, elastase, proelastase or a combination thereof.

The digestive or gastrointestinal protease can be selected from a group of digestive or gastrointestinal proteases released or produced by an exogenous organism or any organism within the digestive tract or released or produced within the digestive tract, e.g., by cells within the tract. In some embodiments, said digestive or gastrointestinal proteases include digestive or gastrointestinal proteases released or produced by an abnormal, infected, cancerous or otherwise diseased tissue.

In an advantageous embodiment of the invention, the antibody or the population of antibodies of the invention has an increased resistance to proteolysis at a pH of about 6 to a pH of about 7,4. Advantageously, the antibody or the population of antibodies of the invention has an increased resistance to proteolysis at a pH of about 6, of about 6,1, of about 6,2, of about 6,3, of about 6,4, of about 6,5, of about 6,6, of about 6,7, of about 6,8, of about 6,9, of about 7,0, of about 7,1, of about 7,2, of about 7,3, of about 7,4. Advantageously, the antibody or the population of antibodies of the invention has an increased resistance to proteolysis which occurs in the small intestine. Advantageously, the antibody or the population of antibodies of the invention has an increased resistance to proteolysis which occurs in the colon. Advantageously, the antibody or the population of antibodies of the invention has an increased resistance to proteolysis which occurs in the small intestine and in the colon.

Measurements of the resistance to proteolysis of the antibody or the population of antibodies of the invention can be accomplished by using standard assays as are known in the prior art, and advantageously by using ELISA and/or flow cytometry. Advantageously, measurements of the resistance to proteolysis of the antibody or the population of antibodies of the invention is performed by flow cytometry.

In a particularly advantageous embodiment of the invention, the residual functional activity of said antibody of the invention in the small intestine, as measured by flow cytometry, is higher than 95% compared with undigested antibody, thirty minutes after the administration of said antibody to the small intestine.

Advantageously, the residual functional activity of said antibody of the invention in the small intestine, as measured by flow cytometry, is higher than 90% compared with undigested antibody, one hour after the administration of said antibody to the small intestine. Advantageously, the residual functional activity of said antibody of the invention in the small intestine, as measured by flow cytometry, is higher than 80% compared with undigested antibody, ninety minutes after the administration of said antibody to the small intestine.

Advantageously, the residual functional activity of said antibody of the invention in the small intestine, as measured by flow cytometry, is higher than 70%, advantageously higher than 75%, compared with undigested antibody, three hour after the administration of said antibody to the small intestine. Advantageously, the residual functional activity of said antibody of the invention in the small intestine, as measured by flow cytometry, is comprised between 70% and 80% compared with undigested antibody, three hours after the administration of said antibody to the small intestine.

Advantageously, the residual functional activity of said antibody of the invention in the small intestine, as measured by flow cytometry, is of 74% compared with undigested antibody, three hours after the administration of said antibody to the small intestine.

In a particularly advantageous embodiment of the invention, the residual functional activity of the population of antibodies of the invention in the ascending colon, as measured by flow cytometry, is higher than 90% compared with undigested population of antibodies, two hours after the administration of said population of antibodies to the ascending colon.

Advantageously, the residual functional activity of said population of antibodies of the invention in the ascending colon, as measured by flow cytometry, is higher than 70%, advantageously higher than 75%, compared with undigested population of antibodies, five hours after the administration of said population of antibodies to the ascending colon. Advantageously, the residual functional activity of said population of antibodies of the invention in the ascending colon, as measured by flow cytometry, is comprised between 70% and 80% compared with undigested population of antibodies, five hours after the administration of said population of antibodies to the ascending colon. Advantageously, the residual functional activity of said population of antibodies of the invention in the ascending colon, as measured by flow cytometry, is of 76% compared with undigested population of antibodies, five hours after the administration of said population of antibodies to the ascending colon.

In a particularly advantageous embodiment of the invention, the residual functional activity of the population of antibodies of the invention in the transverse colon, as measured by flow cytometry, is higher than 80% compared with undigested population of antibodies, two hours after the administration of said population of antibodies to the transverse colon.

Advantageously, the residual functional activity of said population of antibodies of the invention in the transverse colon, as measured by flow cytometry, is higher than 60%, advantageously higher than 65%, compared with undigested population of antibodies, five hours after the administration of said population of antibodies to the transverse colon. Advantageously, the residual functional activity of said population of antibodies of the invention in the transverse colon, as measured by flow cytometry, is comprised between 60% and 70% compared with undigested population of antibodies, five hours after the administration of said population of antibodies to the transverse colon. Advantageously, the residual functional activity of said population of antibodies of the invention in the transverse colon, as measured by flow cytometry, is of 65% compared with undigested population of antibodies, five hours after the administration of said population of antibodies to the transverse colon.

In a particularly advantageous embodiment of the invention, the residual functional activity of the population of antibodies of the invention in the descending colon, as measured by flow cytometry, is higher than 50% advantageously higher than 55%, compared with undigested population of antibodies, two hours after the administration of said population of antibodies to the descending colon.

Advantageously, the residual functional activity of said population of antibodies of the invention in the descending colon, as measured by flow cytometry, is higher than 40%, advantageously higher than 43%, compared with undigested population of antibodies, five hours after the administration of said population of antibodies to the descending colon. Advantageously, the residual functional activity of said population of antibodies of the invention in the descending colon, as measured by flow cytometry, is comprised between 40% and 50% compared with undigested population of antibodies, five hours after the administration of said population of antibodies to the descending colon. Advantageously, the residual functional activity of said population of antibodies of the invention in the descending colon, as measured by flow cytometry, is of 44% compared with undigested population of antibodies, five hours after the administration of said population of antibodies to the descending colon.

In advantageous embodiment of the invention, the antibody or the population of antibodies of the invention is a monoclonal antibody or a population of monoclonal antibodies. According to the present invention, the antibody is not a polyclonal antibody, e.g. the antibody of the invention is not a composition of different antibody molecules, which is capable of binding to or reacting with several different specific antigenic determinants on the same or on different antigens.

In advantageous embodiment of the invention, the antibody or the population of antibodies of the invention can be selected among the group comprising : anti-TNFα antibody (also called anti-TNF alpha antibody), anti-interferon type I receptor (also called anti-IFN I receptor), anti-IL1 antibody, anti-IL6 antibody, anti-IL8 antibody, anti-IL12 antibody, anti-IL13 antibody, anti-IL17 antibody, anti-IL23 antibody, anti-IL34 antibody, anti-CD3 antibody, anti-CD4 antibody, anti-CD20 antibody, anti-CD70 antibody, anti-IFN-γ antibody (also called anti-IFN gamma antibody), anti-INF-γ receptor (also called anti-IFN gamma receptor), anti-CEA antibody, anti-integrin antibody, in particular anti-integrin α antibody or anti-integrin β antibody and advantageously anti-integrin α4β7 antibody, anti-b7 antibody, anti-VEGF antibody and anti-EGFR antibody.

Examples of anti-TNFα antibody can include, but are not limited to adalimumab, infliximab, etanercept, certolizumab, ozoralizumab or golimumab. Examples of anti-IL1 antibody can include, but are not limited to canakinumab. Examples of anti-IL6 antibody can include, but are not limited to tocilizumab, sirukumab, olokizumab and elsilimomab. Examples of anti-IL13 antibody can include, but are not limited to lebrikizumab, dectrekumab and anrukinzumab. Examples of anti- IL17 antibody can include, but are not limited to secukinumab, ixekizumab and brodalumab. Examples of anti-IL23 antibody can include, but are not limited to brazikumab, rizankinumab and birakinumab. Examples of anti-IL12/IL23 antibody can include, but are not limited to ustekinumab. Examples of anti-CD3 antibody can include, but are not limited to visilizumab. Examples of anti-CD4 antibody can include, but are not limited to priliximab. Examples of anti-CD20 antibody can include, but are not limited to rituximab, ofatumumab, ublituximab, obinutuzumab, ocaratuzumab and ocrelizumab. Examples of anti-IFN-gamma antibody can include, but are not limited to fontolizumab. As an example of anti-CEA antibody, can include, but are not limited to labetuzumab. Examples of anti-integrin α4β7 antibody can include, but are not limited to vedolizumab, etrolizumab and abrilumab. Examples of α-integrin antibody can include, but are not limited to natalizumab. Examples of anti-VEGF antibody can include, but are not limited to bevacizumab, and ramucirumab. Examples of anti-EGFR antibody can include, but are not limited to cetuximab and panitumumab.

In a particularly advantageous embodiment of the invention, the antibody or the population of antibodies of the invention is an anti-TNF alpha antibody. In a particularly advantageous embodiment of the invention, the heavy chain of said anti-TNF alpha antibody of the invention comprises the amino acid sequence SEQ ID No: 1 and the light chain of anti-TNF alpha antibody comprises the amino acid sequence SEQ ID No: 2. In a particularly advantageous embodiment of the invention, the anti-TNFα antibody is adalimumab.

In a particularly advantageous embodiment of the invention, the antibody or the population of antibodies of the invention is formulated for oral administration.

According to another aspect of the invention, the present invention concerns a composition comprising the antibody or the population of antibodies as defined above together with at least one pharmaceutically acceptable excipient or carrier. According to the invention, the composition may be used as a medicament. The compositions according to the present invention may be prepared as pharmaceutical compositions.

Within the context of the invention, the term "pharmaceutically acceptable excipient" refers to and includes compounds or materials used to facilitate administration of one or more compounds (or one or more active ingredients), for example, to increase the solubility of the compound. Typical, non-limiting examples of solid excipients include starch, lactose, dicalcium phosphate, sucrose, and kaolin. Typical, non-limiting examples of liquid excipients include sterile water, saline, buffers, non-ionic surfactants, and edible oils. In addition, various adjuvants commonly used in the art may also be included. These and other such compounds are described in literature, e.g., in the Merck Index (Merck & Company, Rahway, N.J.).

According to embodiments that involve administering to a subject in need of treatment a therapeutically effective amount of the composition as provided herein, "therapeutically effective" or "an amount effective to treat" or "pharmaceutically effective" denotes the amount of the composition of the invention needed to inhibit or reverse a disease condition (e.g., to treat an inflammatory disorder or an autoimmune disorder). Determining a therapeutically effective amount specifically depends on such factors as toxicity and efficacy of the medicament. These factors will differ depending on other factors such as potency, relative bioavailability, patient body weight, severity of adverse side-effects and preferred mode of administration. Toxicity may be determined using methods well known in the art. Efficacy may be determined utilizing the same guidance. A pharmaceutically effective amount, therefore, is an amount that is deemed by the clinician to be toxicologically tolerable, yet efficacious.

Dosage may be adjusted appropriately to achieve desired drug (e.g., composition of the invention) levels, local or systemic, depending upon the mode of administration. In the event that the response in a subject is insufficient at such doses, even higher doses (or effective higher doses by a different, more localized delivery route) may be employed to the extent that patient tolerance permits. Multiple doses per day may also be employed to achieve appropriate systemic levels of active compounds. Appropriate systemic levels can be determined by, for example, measurement of the patient's peak or sustained plasma level of the drug. "Dose" and "dosage" are used interchangeably herein.

In some embodiments, the compositions provided are employed for *in vivo* applications. Depending on the intended mode of administration *in vivo* the compositions used may be in the dosage form of solid, semi-solid or liquid such as, e.g., tablets, pills, powders, capsules, gels, ointments, liquids, suspensions, or the like. Preferably, the compositions are administered in unit dosage forms suitable for single administration of precise dosage amounts. The compositions may also include, depending on the formulation desired, at least one pharmaceutically acceptable carrier or diluent, which are defined as aqueous-based vehicles commonly used to formulate pharmaceutical compositions for animal or human administration. The diluent is selected so as not to affect the biological activity of the active compounds present in the combination composition of the invention. Examples of such diluents are distilled water, physiological saline, Ringer's solution, dextrose solution, and Hank's solution. The same diluents may be used to reconstitute a lyophilized antibody of interest. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier are amounts which are effective to obtain a pharmaceutically acceptable formulation in terms of solubility of components, biological activity, etc. In some embodiments the compositions provided herein are sterile.

In one embodiment, the composition herein is administered by oral route.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. The component or components may be chemically modified so that oral delivery of the antibodies is efficacious. Generally, the chemical modification contemplated is the attachment of at least one molecule to the antibodies, where said molecule permits (a) inhibition of proteolysis; and (b) uptake into the blood stream from the stomach or intestine. Also desired is the increase in overall stability of the antibodies and increase in circulation time in the body. For oral compositions, the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the active compounds or by release of the biologically active material beyond the stomach environment, such as in the intestine.

In a particular embodiment, the pharmaceutical composition of the invention as defined above is free of at least one of the following excipients: caprylic acid or a salt of caprylate, glucose, fructose, galactose, mannose, sorbose, sucrose, ribose, deoxyribose, sucrose, maltitol, inulin, lecithin, trehalose, lactose, maltose, raffinose, mannitol, sorbitol, glycerol, arabitol, lactitol, xylitol, polypropylene glycol, polyethylene glycol, polyoxyethylene sorbitan fatty acid ester, polysorbate 20, polysorbate 80, poloxamers, polyoxyethylene alkyl ethers, alkylphenylpolyoxyethylene ethers, polyoxyethylene-polyoxypropylene copolymer, sodium dodecylsulphate, sodium succinate, sodium chloride, sodium citrate, citric acid monohydrate, di-sodium phosphate dibasic hydrated, sodium phosphate monobasic dihydrate, sodium phosphate monobasic monohydrate or EDTA.

For the purposes of the present invention, the term "free" means that the composition of the invention does not contain caprylic acid or salt of caprylate, glucose, fructose, galactose, mannose, sorbose, sucrose , ribose, deoxyribose, sucrose, maltitol, inulin, lecithin, trehalose, lactose, maltose, raffinose, mannitol, sorbitol, glycerol, arabitol, lactitol, xylitol , polypropylene glycol, polyethylene glycol, polyoxyethylene sorbitan fatty acid ester, polysorbate 20, polysorbate 80, poloxamers, polyoxyethylene alkyl ethers, alkylphenylpolyoxyethylene ethers, copolymer of polyoxyethylene-polyoxypropylene, sodium dodecylsulphate, sodium succinate, sodium chloride, sodium citrate, citric acid monohydrate, sodium phosphate dibasic dihydrate, sodium phosphate monobasic dihydrate, ph monobasic sodium monohydrate or EDTA or they are present in a negligible amount, in particular less than 0.001% (w / w).

In a particularly advantageous embodiment, the pharmaceutical composition of the invention as defined above is free of anticoagulant effect molecule, in particular EDTA.

In another particularly advantageous embodiment, the pharmaceutical composition of the invention as defined above is free of surfactant, in particular polysorbate 20, polysorbate 80 or poloxamer.

In another particularly advantageous embodiment, the pharmaceutical composition of the invention as defined above is free of laxative molecule, in particular mannitol, sorbitol, glycerol, lactitol or xylitol.

The excipients mentioned above have in particular been discarded because of their anticoagulant activity such as EDTA, or because they present a risk of developing Crohn's disease when ingested, such as in particular polysorbate 20, the polysorbate 80 or poloxamer, or because these excipients may have a laxative effect, such as in particular mannitol, maltitol, lactitol, xylitol or sorbitol.

In a particularly advantageous embodiment of the invention, the pharmaceutical composition of the invention comprises at least one pharmaceutical-acceptable excipient, which is selected among the group comprising a carboxymethyldextran, a chitosan, a cyclodextrin and a combination thereof.

In one embodiment, the pharmaceutical composition of the invention comprises the antibody or the population of antibodies as defined above as active ingredient and a carboxymethyldextran, as pharmaceutical-acceptable excipient.

In one embodiment, the pharmaceutical composition of the invention comprises the antibody or the population of antibodies as defined above as active ingredient and a chitosan, as pharmaceutical-acceptable excipient.

In one embodiment, the pharmaceutical composition of the invention comprises the antibody or the population of antibodies as defined above as active ingredient and a cyclodextrin, as pharmaceutical-acceptable excipient.

In an advantageous embodiment of the invention, the cyclodextrin may be chosen from α-cyclodextrin, β-cyciodextrin, γ-cyclodextrin, a derivative thereof, such as in particular a hydroxypropyl, hydroxyethyl, ethyl or methyl derivative, a sulfobutyl cyclodextrin beta ether, a branched cyclodextrin, a cyclodextrin-based polymer, or a mixture thereof. Advantageously, the cyclodextrin may be hydroxypropyl-β-cyclodextrin (or HPBCD).

In one embodiment, the pharmaceutical composition of the invention comprises the antibody or the population of antibodies as defined above as active ingredient and a hydroxypropyl-β-cyclodextrin, as pharmaceutical-acceptable excipient.

In another embodiment of the invention, the pharmaceutical composition of the invention comprises the antibody or the population of antibodies as defined above as active ingredient and a combination of a carboxymethyldextran and a cyclodextrin, preferably hydroxypropyl-β-cyclodextrin, as pharmaceutical-acceptable excipient.

In another embodiment of the invention, the pharmaceutical composition of the invention comprises the antibody or the population of antibodies as defined above as active ingredient and a combination of a carboxymethyldextran and a chitosan, as pharmaceutical-acceptable excipient.

Within the context of the invention, the term "combination" refers to a mixture of at least two pharmaceutical-acceptable excipients, advantageously in a single formulation.

In another embodiment of the invention, the pharmaceutical composition of the invention comprises the antibody or the population of antibodies as defined above as active ingredient and a combination of a cyclodextrin, preferably hydroxypropyl-β-cyclodextrin and a chitosan, as pharmaceutical-acceptable excipient.

In another embodiment of the invention, the pharmaceutical composition of the invention comprises the antibody or the population of antibodies as defined above as active ingredient and a combination of a carboxymethyldextran, a cyclodextrin, preferably hydroxypropyl-β-cyclodextrin and a chitosan, as pharmaceutical-acceptable excipient.

In a particularly advantageous embodiment of the invention, the pharmaceutical composition of the invention may further comprise at least one amino acid. For the purposes of the present invention, the amino acid may be chosen from arginine, glycine, lysine, histidine, glutamate, glutamine, asparagine, isoleucine, leucine, alanine, phenylalanine, threonine, tyrosine, tryptophan, methionine, serine, proline, cysteine, selenocysteine, proline, valine, a derivative salt thereof, such as a hydrochloride or a phosphate, or a mixture of these. Advantageously, the amino acid is chosen from hydrophilic amino acids. In an advantageous embodiment, the amino acid is glycine or one of these derived salts, such as glycine hydrochloride. In another particularly advantageous embodiment, the amino acid is arginine or one of these derived salts, such as arginine hydrochloride.

In a particularly advantageous embodiment of the invention, the pharmaceutical composition comprises the antibody or the population of antibodies as defined above as active ingredient, a carboxymethyldextran, as pharmaceutical-acceptable excipient, and an amino acid, in particular glycine, optionally in its hydrochloride form.

In a particularly advantageous embodiment of the invention, the pharmaceutical composition comprises the antibody or the population of antibodies as defined above as active ingredient, a chitosan, as pharmaceutical-acceptable excipient, and an amino acid, in particular glycine, optionally in its hydrochloride form.

In a particularly advantageous embodiment of the invention, the pharmaceutical composition comprises the antibody or the population of antibodies as defined above as active ingredient, a cyclodextrin, preferably hydroxypropyl-β-cyclodextrin, as pharmaceutical-acceptable excipient, and an amino acid, in particular glycine, optionally in its hydrochloride form.

In another embodiment of the invention, the pharmaceutical composition of the invention comprises the antibody or the population of antibodies as defined above as active ingredient and a combination of a carboxymethyldextran and a cyclodextrin, preferably hydroxypropyl-β-cyclodextrin, as pharmaceutical-acceptable excipient and an amino acid, in particular glycine, optionally in its hydrochloride form.

In another embodiment of the invention, the pharmaceutical composition of the invention comprises the antibody or the population of antibodies as defined above as active ingredient and a combination of a carboxymethyldextran and a chitosan, as pharmaceutical-acceptable excipient and an amino acid, in particular glycine, optionally in its hydrochloride form.

In another embodiment of the invention, the pharmaceutical composition of the invention comprises the antibody or the population of antibodies as defined above as active ingredient and a combination of a cyclodextrin, preferably hydroxypropyl-β-cyclodextrin and a chitosan, as pharmaceutical-acceptable excipient and an amino acid, in particular glycine, optionally in its hydrochloride form.

In another embodiment of the invention, the pharmaceutical composition of the invention comprises the antibody or the population of antibodies as defined above as active ingredient and a combination of a carboxymethyldextran, a cyclodextrin, preferably hydroxypropyl-β-cyclodextrin and a chitosan, as pharmaceutical-acceptable excipient and an amino acid, in particular glycine, optionally in its hydrochloride form.

In a particularly advantageous embodiment of the invention, the pharmaceutical composition of the invention consists of the antibody or the population of antibodies as defined above as active ingredient and a carboxymethyldextran, as pharmaceutical-acceptable excipient.

In a particularly advantageous embodiment of the invention, the pharmaceutical composition of the invention consists of the antibody or the population of antibodies as defined above as active ingredient and a carboxymethyldextran and cyclodextrin, preferably hydroxypropyl-β-cyclodextrin, as pharmaceutical-acceptable excipient.

In a particularly advantageous embodiment of the invention, the pharmaceutical composition of the invention consists of the antibody or the population of antibodies as defined above as active ingredient and a carboxymethyldextran and cyclodextrin, preferably hydroxypropyl-β-cyclodextrin, as pharmaceutical-acceptable excipient, and an amino acid, in particular glycine, optionally in its hydrochloride form.

In a particularly advantageous embodiment of the invention, the pharmaceutical composition of the invention consists of the antibody or the population of antibodies as defined above as active ingredient and a chitosan, as pharmaceutical-acceptable excipient.

In a particularly advantageous embodiment of the invention, the composition of the invention is administered orally to the gastrointestinal tract (GI).

Within the context of the invention, the term "gastrointestinal tract" or "GI tract" is understood to include the stomach, small intestine (duodenum, jejunum, ileum), large intestine (cecum, colon, rectum) and anus.

In a particularly advantageous embodiment of the invention, the composition of the invention is administered in a dosage form suitable for oral administration to the gastrointestinal tract (GI).

Another subject of the invention relates to the use of a pharmaceutical composition comprising a therapeutically effective amount of antibody or the population of antibodies as defined above as an active ingredient in the manufacture of a medicinal product intended for the prevention and/or the treatment of inflammatory disorder or inflammatory disease.

In a particular advantageous embodiment, the present invention relates to the use of a pharmaceutical composition comprising a therapeutically effective amount of antibody or the population of antibodies as defined above as an active ingredient in the manufacture of a medicinal product intended for the prevention and/or the treatment of autoimmune disorders.

Within the context of the invention, the term "autoimmune disease and inflammatory disorders" refers to rheumatoid arthritis, psoriasis, Crohn's disease, ankylating spondylitis, ulcerative colitis, chronic inflammatory bowel disease (IBD), irritable bowel syndrome (IBS), Crohn's disease, ulcerative colitis, bleeding rectal ulcer, concomitant lesions of Behget's disease, pouchitis and cancer in particular cancer of the small intestine and colorectal cancer.

Another subject of the invention relates to a method for preventing and/or treating inflammatory disorders or inflammatory disease in patients in need of, comprising the administration to said patients of the antibody or the population of antibodies of the invention or a pharmaceutical composition comprising a therapeutically effective amount of the antibody or the population of antibodies of the invention as an active ingredient and at least one pharmaceutically acceptable excipient as defined above.

Another subject of the invention relates to a method for preventing and/or treating autoimmune disorders in patients in need of, comprising the administration to said patients of the antibody or the population of antibodies of the invention or a pharmaceutical composition comprising a therapeutically effective amount of the antibody or the population of antibodies of the invention as an active ingredient and at least one pharmaceutically acceptable excipient as defined above.

### FIGURES

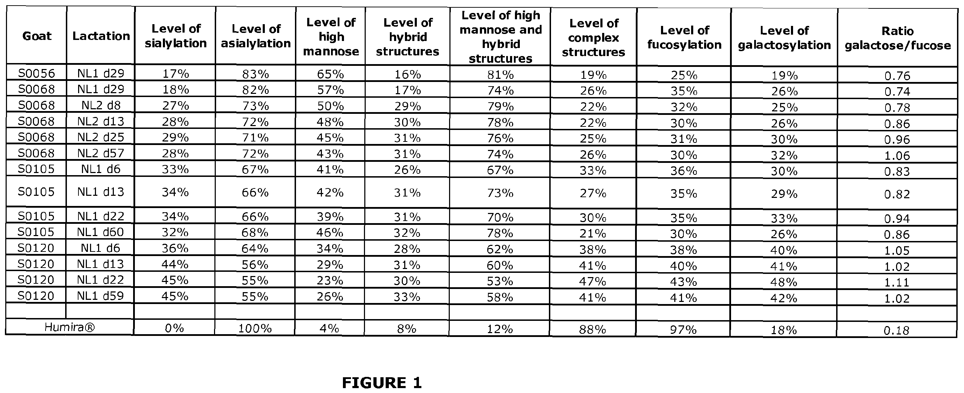

**Figure 1** shows the glycosylation pattern of the antibody of the invention (anti-TNF alpha antibody which has been produced in the milk of transgenic goat and which comprises the amino acid sequence SEQ ID No: 1 as heavy chain and the amino acid sequence SEQ ID No: 2 as light chain) and a commercially available anti-TNF alpha antibody Humira® (adalimumab). The level of each motif is expressed in percentage compared to the total of glycan forms bound to N-glycosylation sites of a population of antibodies. The term "d" refers to the day of lactation. The term "NL1" refers to New Lactation 1 and the term "NL2" refers to New Lactation 2.

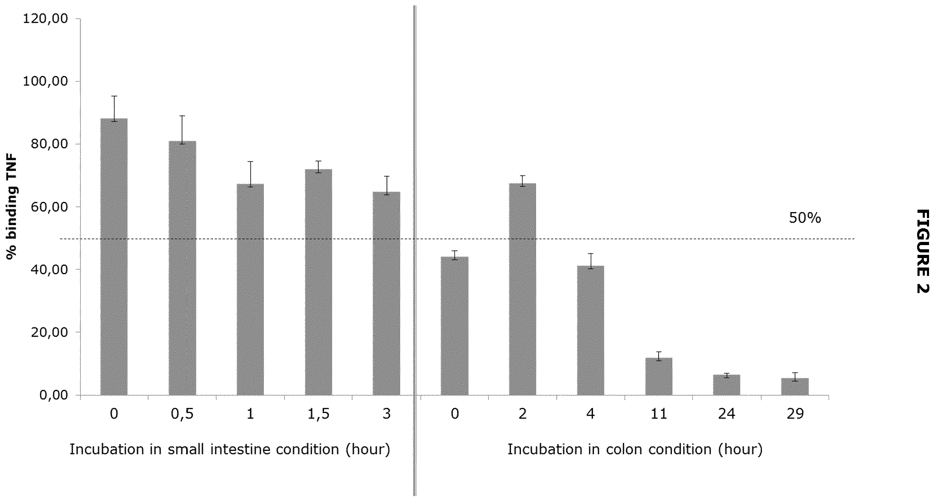

**Figure 2** concerns the ability of the anti-TNF alpha antibody of the invention to bind soluble TNF-alpha, after incubation in the small intestine and in the colon. The ability of the anti-TNF alpha antibody of the invention to bind soluble TNF-alpha has been measured by ELISA. The anti-TNF alpha antibody of the invention has been produced in the milk of transgenic goat and comprises the amino acid sequence SEQ ID No: 1 as heavy chain and the amino acid sequence SEQ ID No: 2 as light chain.

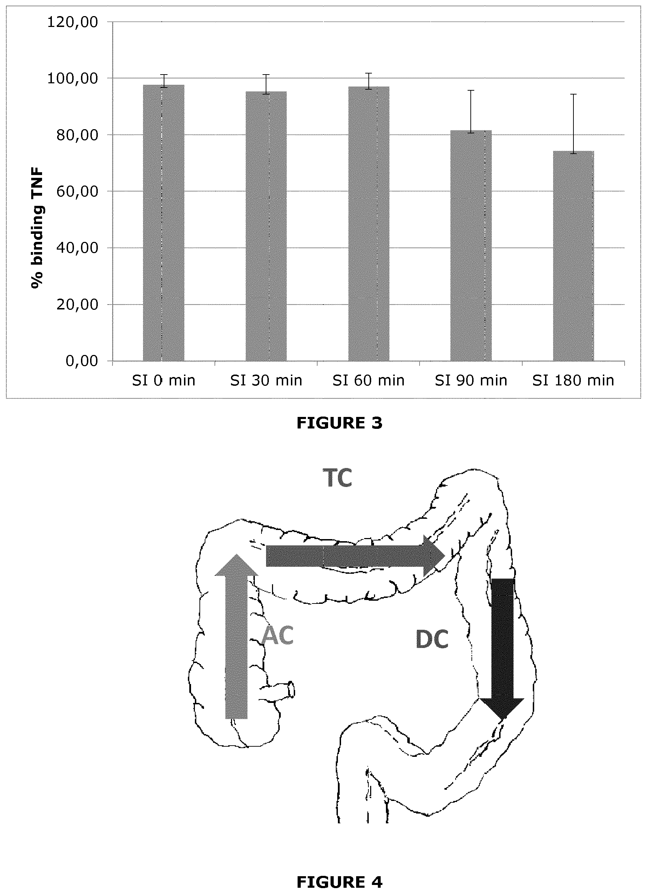

**Figure 3** concerns the ability of the anti-TNF alpha antibody of the invention to bind soluble TNF-alpha, after incubation in the small intestine. The ability of the anti-TNF alpha antibody of the invention to bind soluble TNF-alpha has been measured by flow cytometry. The anti-TNF alpha antibody of the invention has been produced in the milk of transgenic goat and comprises the amino acid sequence SEQ ID No: 1 as heavy chain and the amino acid sequence SEQ ID No: 2 as light chain.
**Figure 4** represents the colon compartments, which comprises the ascending colon (AC), the transverse colon (TC) and the descending colon (DC).

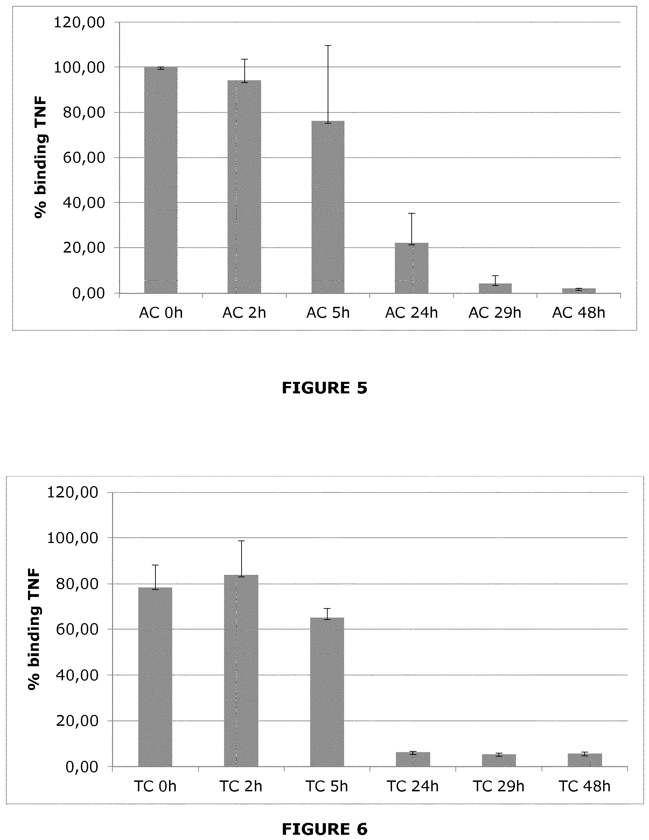

**Figure 5** concerns the ability of the anti-TNF alpha antibody of the invention to bind soluble TNF-alpha, after incubation in the ascending colon (AC). The ability of the anti-TNF alpha antibody of the invention to bind soluble TNF-alpha has been measured by flow cytometry. The anti-TNF alpha antibody of the invention has been produced in the milk of transgenic goat and comprises the amino acid sequence SEQ ID No: 1 as heavy chain and the amino acid sequence SEQ ID No: 2 as light chain.
**Figure 6** concerns the ability of the anti-TNF alpha antibody of the invention to bind soluble TNF-alpha, after incubation in the transverse colon (TC). The ability of the anti-TNF alpha antibody of the invention to bind soluble TNF-alpha has been measured by flow cytometry. The anti-TNF alpha antibody of the invention has been produced in the milk of transgenic goat and comprises the amino acid sequence SEQ ID No: 1 as heavy chain and the amino acid sequence SEQ ID No: 2 as light chain.

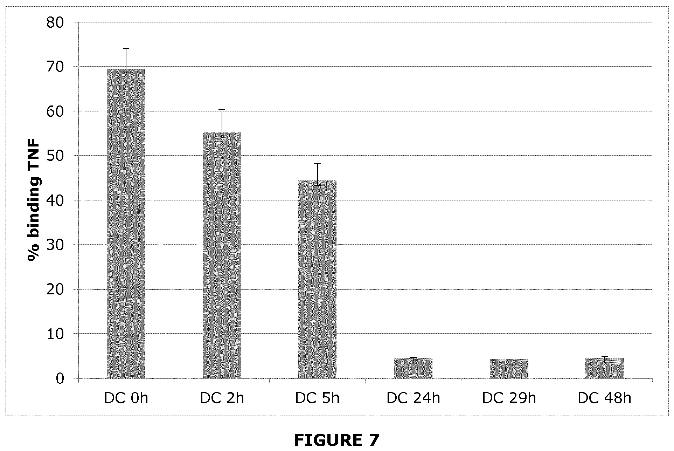

**Figure 7** concerns the ability of the anti-TNF alpha antibody of the invention to bind soluble TNF-alpha, after incubation in the descending colon (DC). The ability of the anti-TNF alpha antibody of the invention to bind soluble TNF-alpha has been measured by flow cytometry. The anti-TNF alpha antibody of the invention has been produced in the milk of transgenic goat and comprises the amino acid sequence SEQ ID No: 1 as heavy chain and the amino acid sequence SEQ ID No: 2 as light chain.

### EXEMPLES

### Example 1: Generation of transgenic goats that produce the antibody of the invention (anti-TNF alpha antibody).

Transgenic goats were generated that include the nucleic acid sequence encoding the anti-TNF alpha antibody which comprises the amino acid sequence SEQ ID No: 1 as heavy chain and the amino acid sequence SEQ ID No: 2 as light chain in their genome. The goats producing anti-TNF alpha antibody were generated using traditional microinjection techniques (See e.g., US 7,928,064). The cDNA encoding the heavy and light chain (SEQ ID No:3 and SEQ ID No:4) was synthesized based on the published amino acid sequence (US Patent 6,090,382). These DNA sequences were ligated with the beta casein expression vector to yield constructs BC2601 HC and BC2602 LC. In these plasmids, the nucleic acid sequence encoding anti-TNF alpha antibody is under the control of a promoter facilitating the expression of anti-TNF alpha antibody in the mammary gland of the goats. The prokaryotic sequences were removed and the DNA microinjected into pre-implantation embryos of the goat. These embryos were then transferred to pseudo pregnant females. The progeny that resulted were screened for the presence of the transgenes. Those that carried both chains were identified as transgenic founders.

When age appropriate, the founder animals were bred. Following pregnancy and parturition they were milked. The time course was in days starting lactation after parturition (e.g., day 3, day 7, day 11). The anti-TNF alpha antibody was purified from the milk at each time point and characterized as described herein.

### Example 2: Comparison of the glycosylation pattern of the antibody of the invention (anti-TNF alpha antibody) and a commercially available anti-TNF alpha antibody (Humira® - adalimumab).

The glycosylation pattern of the anti-TNF alpha antibody of the invention produced in the milk of transgenic goats and the glycosylation pattern of Humira® were determined by releasing the N-glycans from antibody and running the released oligosaccharides on a column ("oligosaccharide signature").

The results are presented in Figure 1.

### Example 3: Evaluation of the resistance of the anti TNF-alpha antibody of the invention in the digestive tract

### 1. Stability studies in simulated gastrointestinal fluids

The stability of the antibody was assessed in simulated gastrointestinal fluids corresponding to small intestine (SI), ascending colon (AC), transverse colon (TC) and descending colon (DC) compartments over a period of 48h with frequent sampling. The experiments consisted of non-sequential, individual, incubations in each of the four different gastrointestinal fluids mentioned.

These four gastrointestinal compartments represent small scale versions of the reactors from the Simulator of the Human Intestinal Microbial Ecosystem (SHIME®) developed by Prodigest (Molly K. et al Appl. Microbiol. Biotechnol., 1993, 39:254-258; Van den Abbeele P. et al Appl. Environ. Microbiol., 2010, 76: 5237-5246).

The Active Pharmaceutical Ingredient (API) tested was the anti-TNF alpha antibody of the invention (batch #052314A1) in 10 mM acetate buffer pH 5.5 (concentration adjusted to 10 mg/ml). The anti-TNF alpha antibody of the invention has the same amino acid sequence as adalimumab but is produced in the milk of transgenic goats.

### A. Procedure for simulated small intestine (SI) compartment

### Preparation of the simulated small intestine (SI) fluid (in triplicates SI1, SI2, SI3):

For preparing the simulated small intestine (SI) fluid, 14 ml of SHIME feed composition with pepsin (2 g/L) and 6 ml of pancreatic juice were introduced in falcon tube.

The SHIME feed composition is composed of (in grams/liter): arabinogalactan (1.0 g/L), pectin (2.0 g/L), xylan (1.0 g/L), starch (4.0 g/L), glucose (0.4 g/L), yeast extract (3.0 g/L), peptone (3.0 g/L), mucin (1.0 g/L), and cystein (0.5 g/L).

The pancreatic juice is composed of 12.5 g NaHCO₃, 6 g dehydrated bile extract (Oxgall, Difco) and 0.9 g porcine pancreatin (Sigma Aldrich, P1625) per liter. Pancreatin contains enzymes produced by the exocrine cells of the porcine pancreas including trypsin, amylase, lipase, ribonuclease and protease.

### Addition of the anti-TNF alpha antibody of the invention and incubation:

700 µL of anti-TNF alpha antibody of the invention stock solution (10 mg/ml) was added to the simulated small intestine fluid. Final anti-TNF alpha antibody of the invention concentration in simulated SI fluid was 350 µg/ml.

The anti-TNF alpha antibody of the invention in simulated small intestine fluid were incubated at 37°C.

### Sampling:

Sampling was performed at 0 hour, 30min, 60min, 90min and 180min.

Each sample are prepared in an Eppendorf tube and comprises 2 ml of the anti-TNF alpha antibody of the invention in simulated small intestine fluid. Aliquots of 200 µl is dosed from the sample.13.4 µl Pefabloc (9 mg/ml) and 26.7 µl Pepstatin (3.082 mg/l) (proteins inhibitors) were added to the aliquots. The aliquots were incubated in a shaking incubator (90 rpm) at 37°C.

### B. Procedure for simulated ascending colon (AC), transverse colon (TC) and descending colon (DC) compartments

### Preparation of the simulated colon fluids (triplicates):

Ascending colon (AC), transverse colon (TC), and descendant colon (DC) fluids were prepared separately.

For each ascending colon (AC), transverse colon (TC), and descendant colon (DC) fluids, 32.6 ml SHIME feed with pepsin (2 g/L) was mixed to 14 ml pancreatic juice and 100.1 mL basal medium (BM) The pancreatic juice is composed of 12.5 g NaHCO3, 6 g dehydrated bile extract (Oxgall, Difco) and 0.9 g porcine pancreatin (Sigma Aldrich, P1625) per liter. Pancreatin contains enzymes produced by the exocrine cells of the porcine pancreas including trypsin, amylase, lipase, ribonuclease and protease. The pH was then measured and adjusted to 5.9 for ascending colon (AC), to 6.4 for transverse colon (TC), and to 6.8 for descendant colon (DC).

### Preparation of penicillin bottles.

Triplicates of simulated ascending colon fluid (AC1, AC2 and AC3) were prepared by introducing 50mL of simulated ascending colon fluid in an autoclaved penicillin bottles.
Triplicates of simulated transverse colon fluid (TC1, TC2 and TC3) were prepared by introducing 50mL of simulated transverse colon fluid in an autoclaved penicillin bottles.
Triplicates of simulated descending colon fluid (DC1, DC2 and DC3) were prepared by introducing 50mL of simulated descending colon fluid in an autoclaved penicillin bottles.
Control was prepared by spiking 150µl of the anti-TNF alpha antibody of the invention in 15ml of PO₄- buffer (0.2M), pH 7.

The bottles were made anaerobic by flushing them with N₂, and they were continuously stirred in a shaking incubator (90 rpm) and kept at 37°C.

### Addition of the anti-TNF alpha antibody of the invention:

500 µL of anti-TNF alpha antibody of the invention stock solution (10 mg/ml) was added to each of the 9 penicillin bottles. Final anti-TNF alpha antibody of the invention concentration in AC/TC/DC colon is 100 µg/ml.

### Sampling:

Sampling was performed at 0 hour, 2 hours, 5 hours, 24 hours, 29 hours and 48 hours.

Each sample are prepared in an Eppendorf tube and comprises 2 ml of the anti-TNF alpha antibody of the invention in simulated small intestine fluid. The sample is than centrifuged at 5400rcf during 10 min. Aliquots of 200 µl is dosed from the sample.13.4 µl Pefabloc (9 mg/ml) and 26.7 µl Pepstatin in DMSO (3.082 mg/l) (proteins inhibitors) were added to the aliquots. The aliquots were incubated in a shaking incubator (90 rpm) at 37°C.

### 2. Evaluation of the binding to soluble TNF-alpha by ELISA

Plates were coated during one night with mouse anti-human TNF-α (ELISA kit Human TNF-α Duoset ELISA R&D system) at 4µg/ml in PBS. After washing and saturation in PBS + 1% BSA, soluble TNF at 100 ng/ml was incubated during 90 minutes at room temperature. After washing, the anti-TNF alpha antibody of the invention were incubated at 30 ng/ml and then detected with an anti-human IgG (H+L) antibody linked to peroxidase.

The peroxidase activity was then measured by coloration of 2,2'-azino-bis (3-ethylbenzthiazoline-6-sulfonic acid.

A standard curve was made with Tg-Humira 052314 A1 from 60 ng/ml to 1 ng/ml.

The sample concentrations obtained with the standard curve were compared to the theoretical concentration to obtain the percentage of TNF fixation.

The results obtained are presented in Figure 2.

These results indicate that:
- one hour after the administration of the anti-TNF alpha antibody of the invention in the small intestine, the functional activity of said antibody in the small intestine, is higher than 70% compared with undigested antibody.
- three hours after the administration of the anti-TNF alpha antibody of the invention in the small intestine, the functional activity of said antibody in the small intestine, is higher than 60% compared with undigested antibody.
- four hours after the administration of the anti-TNF alpha antibody of the invention in the colon, the functional activity of said antibody in the colon, is higher than 40% compared with undigested antibody.

### 3. Residual functional activity assessment bv flow cytometry

Residual functional activity of the anti-TNF alpha antibody of the invention during gastrointestinal fluid incubation was assessed by measuring the binding of said antibody to membrane TNF-alpha in a cell-base assay, by flow cytometry.

### Reagents

▪ Antibody of the invention: anti-TNF alpha antibody produced in the milk of transgenic goats
▪ Goat anti-human IgG Fc coupled to phycoerythrin (Beckman Coulter) was used for antibody detection by flow cytometry.
▪ Cells: Jurkat cells expressing membrane TNF-alpha at their surface were obtained by transfection with an expression vector expressing a TNF-alpha mutant without soluble TNF cleavage site.

### Binding to membrane TNF-alpha by flow cytometry

Anti-TNF alpha antibodies of the invention was tested at different concentrations (5, 1, 0.5, 0.25, 0.125, 0.06, 0.03, 0.015 µg/ml). Gastrointestinal fluids samples were diluted at a 0,125µg/ml theoretical concentration. 2 x 10⁵ TNFα transfected Jurkat cells (100µl) were incubated with 100 µl of anti-TNF alpha antibody of the invention at different concentration (5, 1, 0.5, 0.25, 0.125, 0.06, 0.03, 0.015 µg/ml) at 4°C for 30 minutes.

After washing, the goat anti-human Fc gamma coupled to phycoerythrin (100 µl of a dilution of 1:100) was added at 4°C and incubated for 30 minutes. The cells were washed and mean of fluorescence intensity (MFI) studied by flow cytometry. Arbitrary Kd was calculated using PRISM software.
Using the standard curve dose response, results were expressed in µg/ml or in percentage of binding, 100% corresponding to the concentration of 0.125µg/ml.

The results obtained are presented in Figures 3 and 5 to 7.

These results indicate that:
- one hour after the administration of the anti-TNF alpha antibody of the invention in the small intestine, the functional activity of said antibody in the small intestine, is of 95% compared with undigested antibody (Figure 3).
- three hours after the administration of the anti-TNF alpha antibody of the invention in the small intestine, the functional activity of said antibody in the small intestine, is of 74% compared with undigested antibody (Figure 3).
- five hours after the administration of said antibody to the ascending colon, the residual functional activity of said antibody in the ascending colon, as measured by flow cytometry, is of 76% compared with undigested antibody (Figure 5).
- five hours after the administration of said antibody to the transverse colon, the residual functional activity of said antibody in the transverse colon, as measured by flow cytometry, is of 65% compared with undigested antibody (Figure 6).
- five hours after the administration of said antibody to the descending colon, the residual functional activity of said antibody in the descending colon, as measured by flow cytometry, is of 44% compared with undigested antibody (Figure 7).

## Claims

1. An antibody produced in mammary epithelial cells, wherein said antibody has an increased resistance to proteolysis by one or more digestive or gastrointestinal proteases.

2. The antibody according to claim 1, wherein said antibody is produced in mammary epithelial cells of a non-human animal or in the milk of a transgenic non-human animal.

3. The antibody according to any one of claims 1 to 2, wherein said antibody has an increased resistance to proteolysis by pepsin, pancreatin, trypsin, chymotrypsin, carboxypepsidase, procarboxypepsidase, elastase, proelastase or a combination thereof.

4. The antibody according to any one of claims 1 to 3, wherein said antibody has an increased resistance to proteolysis at a pH of about 6 to a pH of about 7,4.

5. The antibody according to any one of claims 1 to 4, wherein one hour after the administration of said antibody to the small intestine, the residual functional activity of said antibody in the small intestine, as measured by flow cytometry, is higher than 90% compared with undigested antibody.

6. The antibody according to any one of claims 1 to 5, wherein three hours after the administration of said antibody to the small intestine, the residual functional activity of said antibody in the small intestine, as measured by flow cytometry, is higher than 70% compared with undigested antibody.

7. The antibody according to any one of claims 1 to 6, wherein five hours after the administration of said antibody to the ascending colon, the residual functional activity of said antibody in the ascending colon, as measured by flow cytometry, is higher than 70% compared with undigested antibody.

8. The antibody according to any one of claims 1 to 7, wherein five hours after the administration of said antibody to the transverse colon, the residual functional activity of said antibody in the transverse colon, as measured by flow cytometry, is higher than 60% compared with undigested antibody.

9. The antibody according to any one of claims 1 to 8, wherein five hours after the administration of said antibody to the descending colon, the residual functional activity of said antibody in the descending colon, as measured by flow cytometry, is higher than 40% compared with undigested antibody.

10. The antibody according to any one of claims 1 to 9, wherein the transgenic non-human animal is goat.

11. The antibody according to any one of claims 1 to 10, wherein said antibody is a monoclonal antibody.

12. The antibody according to any one of claims 1 to 11, wherein said antibody is an anti-TNF alpha antibody.

13. The antibody according to claim 12, wherein the heavy chain of said anti-TNF alpha antibody comprises the amino acid sequence SEQ ID No: 1 and the light chain of anti-TNF alpha antibody comprises the amino acid sequence SEQ ID No: 2.

14. The antibody according to any one of claims 1 to 13, wherein the antibody is formulated for oral administration.

15. A composition comprising the antibody of claims 1 to 14 and at least one pharmaceutically-acceptable excipient or carrier.

16. The composition according to claim 15, wherein the at least one pharmaceutical-acceptable excipient is selected among the group comprising a carboxymethyldextran, a chitosan, a cyclodextrin and a combination thereof.

17. The composition according to any one of claims 15 to 16, wherein the composition is administered orally to the gastrointestinal tract (GI).

18. The composition according to any one of claims 15 to 17, wherein the composition is administered in a dosage form suitable for oral administration to the gastrointestinal tract (GI).

19. The composition as defined in any one of claims 15 to 18, for its use in preventing or treating an inflammatory disorder or an autoimmune disorder.


SEQUENCE LISTING

<110>  Laboratoire Français du Fractionnement et des
       Biotechnologies

<120>  ANTIBODIES WITH INCREASED ACTIVITY IN THE DIGESTIVE TRACT

<130>  IOBV18LFBTNF

<160>  4

<170>  PatentIn version 3.5

<210>  1
<211>  470
<212>  PRT
<213>  Homo sapiens

<400>  1

Met Glu Phe Gly Leu Ser Trp Leu Phe Leu Val Ala Ile Leu Lys Gly
1               5                   10                  15

Val Gln Cys Glu Val Gln Leu Val Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Asp Asp Tyr Ala Met His Trp Val Arg Gln Ala Pro Gly Lys Gly Leu
    50                  55                  60

Glu Trp Val Ser Ala Ile Thr Trp Asn Ser Gly His Ile Asp Tyr Ala
65                  70                  75                  80

Asp Ser Val Glu Gly Arg Phe Thr Ile Ser Arg Asp Asn Ala Lys Asn
                85                  90                  95

Ser Leu Tyr Leu Gln Met Asn Ser Leu Arg Ala Glu Asp Thr Ala Val
            100                 105                 110

Tyr Tyr Cys Ala Lys Val Ser Tyr Leu Ser Thr Ala Ser Ser Leu Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys
465                 470

<210>  2
<211>  236
<212>  PRT
<213>  Homo sapiens

<400>  2

Met Asp Met Arg Val Pro Ala Gln Leu Leu Gly Leu Leu Leu Leu Trp
1               5                   10                  15

Leu Arg Gly Ala Arg Cys Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Gly Ile Arg Asn Tyr Leu Ala Trp Tyr Gln Gln Lys Pro Gly Lys
    50                  55                  60

Ala Pro Lys Leu Leu Ile Tyr Ala Ala Ser Thr Leu Gln Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Pro Glu Asp Val Ala Thr Tyr Tyr Cys Gln Arg
            100                 105                 110

Tyr Asn Arg Ala Pro Tyr Thr Phe Gly Gln Gly Thr Lys Val Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235

<210>  3
<211>  1416
<212>  DNA
<213>  Homo sapiens

<400>  3
atggaattcg gcctgagctg gctgttcctg gtggccatcc tgaagggcgt gcagtgcgag      60

gtgcagctgg tggagtctgg cggaggactg gtgcagcccg gcagaagcct gagactgagc     120

tgcgccgcca gcggcttcac cttcgacgac tacgccatgc actgggtccg ccaggcccct     180

ggaaagggcc tggaatgggt gtccgccatc acctggaaca gcggccacat cgactacgcc     240

gacagcgtgg agggcaggtt caccatcagc agggacaacg ccaagaacag cctgtacctg     300

cagatgaaca gcctgagggc cgaggacacc gccgtgtact actgcgccaa ggtgtcctac     360

ctgagcaccg ccagcagcct ggattactgg ggccagggca ccctggtgac cgtgtccagc     420

gccagcacca agggccctag cgtgttccct ctggccccca gcagcaagtc tacctctggc     480

ggcacagccg ctctgggctg cctggtgaag gactacttcc ccgagcccgt gacagtgtcc     540

tggaactctg gcgccctgac cagcggcgtg cacacattcc ctgccgtgct gcagagcagc     600

ggcctgtaca gcctgagcag cgtggtgaca gtgcctagca gctctctggg cacccagacc     660

tacatctgca acgtgaacca caagcccagc aacaccaagg tggacaagaa ggtggagccc     720

aagagctgcg acaagaccca cacctgtccc ccttgtcctg cccctgagct gctgggcgga     780

cccagcgtgt tcctgttccc cccaaagccc aaggacaccc tgatgatcag caggaccccc     840

gaagtgacct gcgtggtggt ggacgtgtcc cacgaggacc ctgaagtgaa gttcaattgg     900

tacgtggacg gcgtggaggt gcacaatgcc aagaccaagc ccagagagga acagtacaac     960

agcacctaca gggtggtgtc cgtgctgacc gtgctgcacc aggactggct gaacggcaaa    1020

gagtacaagt gcaaggtctc caacaaggcc ctgcctgccc ccatcgagaa aaccatcagc    1080

aaggccaagg gccagcccag agaaccccag gtgtacaccc tgccccctag cagggacgag     1140

ctgaccaaga accaggtgtc cctgacctgt ctggtgaagg gcttctaccc cagcgatatc    1200

gccgtggagt gggagtctaa cggccagcct gagaacaact acaagaccac cccccctgtg 1260

ctggacagcg acggcagctt cttcctgtac tccaaactga ccgtggacaa gagcagatgg 1320

cagcagggca acgtgttcag ctgcagcgtg atgcacgagg ccctgcacaa ccactacacc 1380

cagaagtccc tgagcctgag ccccggcaag taatga 1416

<210> 4
<211> 714
<212> DNA
<213> Homo sapiens

<400> 4
atggacatga gagtgcccgc tcagctgctg ggactgctgc tgctgtggct gagaggcgcc 60

agatgcgaca tccagatgac ccagagccct tctagcctga gcgccagcgt gggcgacaga 120

gtgaccatca cctgtagggc cagccagggc atcaggaact acctggcctg gtatcagcag 180

aagcccggca aggcccccaa gctgctgatc tacgccgcca gcaccctgca gagcggcgtg 240

cccagcagat tcagcggcag cggctccggc accgacttca ccctgaccat cagcagcctg 300

cagcctgagg acgtggccac ctactactgc cagaggtaca acagggcccc ctacaccttc 360

ggacagggca ccaaggtgga gatcaagagg accgtggccg ctcccagcgt gttcatcttc 420

ccacccagcg acgagcagct gaagtctggc accgcctccg tggtctgcct gctgaacaac 480

ttctaccccc gcgaggccaa ggtgcagtgg aaggtggaca acgccctgca gtccggcaac 540

agccaggaaa gcgtcaccga gcaggacagc aaggactcca cctactccct gtccagcacc 600

ctgaccctga gcaaggccga ctacgagaag cacaaggtgt acgcctgcga agtgacccac 660

cagggcctga gcagccctgt gaccaagagc ttcaacaggg gcgagtgcta atga 714